# EUROPEAN PATENT APPLICATION

(11) **EP 0 629 649 A1**
(43) Date of publication of application: **21.12.1994**
(21) Application number: 94401326.7
(22) Date of filing: 13.06.1994
(51) Int. Cl.: C08G 81/02, E21B 43/22, E21B 43/25, E21B 43/26, E21B 43/27, E21B 33/13, C10M 173/02, C09K 7/02

(54) **Rheofluidifying polymers, their synthesis and their applications particularly in the oil industry**

(30) Priority: 16.06.1993 FR 9307356
(71) Applicant: SOFITECH N.V., Uccle (1180 Bruxelles) (BE)
(72) Inventor: Maroy, Pierre, F-78530 Buc (FR); L'Alloret, Florence, F-75012 Paris (FR); Hendriks, Hugo, F-38100 Grenoble (FR); Hourdet, Dominique, F-94360 Bry Sur Marne (FR)
(74) Representative: Hagel, Francis

(57) **Abstract**

The invention relates to polymers the aqueous solutions of which have a rheofluidifying behaviour that increases with temperature.

These polymers comprise a water-soluble skeleton incorporating segments or bearing side chains having a Lower Critical Solution Temperature ("LCST" property).
Applications to the chemical and oil industries, in particular for their controlled anti-settling properties and, most especially, to the field of drilling muds, as well as other technical sectors using this property, such as cosmetics, the food processing industry or any other industry in which there arise problems relating to the stability of a suspension in an aqueous solution of solid particles having sizes of less than a few millimeters.

## Description

The present invention relates to the technical sector concerned with polymers and, more particularly, rheofluidifying polymers and copolymers whose solutions in an aqueous medium have a rheofluidifying behaviour that increases with temperature.

"Rheofluidifying behaviour" designates the property whereby measured apparent viscosity decreases when the shearing force applied to the fluid becomes stronger.

In this context, the expression "increases" means that links are associated locally above a certain threshold temperature, which causes a physical increase in molecular weight which is more or less reduced by the level of shearing applied to the solution. As aggregation increases with a rise in temperature, the rheofluidifyig nature of aqueous solutions is thus all the more marked the higher the temperature.

As used in this context, the expression "increases" will assume these two meanings throughout the text.

Such products have numerous applications as antisettling agents, as components of fracturing fluids or drilling or completion fluids, in particular for well cementing, in the oil and geothermal industries, or acid well treatment, as well as of mobility control agents or fluids for enhanced oil recovery (EOR), and analogous fluids used in the oil industry, such as industrial cleaning fluids or aqueous base hydraulic lubricating fluids.

The very great majority of these applications take place in an aqueous medium. One could, however, upon reading the following description, contemplate choosing polymers usable in a non-aqueous medium; mention can be made, in particular, to gels for industrial cleaning, in an alcohol medium, in particular in ethanol. Their field of application thus covers a very wide range of industries and their potential uses will become clear to a man of the art in the light of the properties described herebelow.

In this field, there is known patent USP 4 432 881, which relates to thickening agents constituted by a water-soluble polymer chain bearing hydrophobic side chains not having an LCST property (as defined below) in the useful temperature range.

On these hydrophobic side chains is fixed the hydrophobic fraction of a surfactant.

When the temperature rises, the surfactant is dehydrated, separates from the water, and a slight increase in viscosity occurs.

In the present application, "useful temperature range", or an analogous expression, will designate the temperature range that corresponds to the application in view for the polymer giving rheofluidifying solutions, which will naturally have to correspond to the temperature range around the LCST range of the links that are subjected thereto.

The invention described in French patent application No. 92 10224, filed on 20th. August 1992, is the result of an approach that is original and inherently simple, in its principle, which consists in fixing on (or incorporating in) a polymer chain, water-soluble in the temperature range of the application under consideration, an appropriate proportion of hydrophilic side chains possessing an LCSTproperty, becoming hydrophobic when the temperature rises(hereinafter referred to as "heat-sensitive side chains").

It has been discovered, according to the present application, that, generally speaking, the said heat-sensitive chains could be appropriately fixed on - or incorporated in - the "macromolecular" structure under consideration from certain prepolymers or macromonomers having a so-called "Lower Critical Solution Temperature" (or LCST) property.

This general principle can be implemented, in a highly preferred manner, according to the two so-called "grafting" or "copolymerization" variants herebelow.

The first preferred approach consists in using known techniques to graft heat-sensitive side chains onto a polymer skeleton, which is, itself, water-soluble. In this case, the side chain has to be provided with an active site capable of cooperating, for grafting purposes, with a corresponding active site on the polymer skeleton.

The second preferred approach consists in using known techniques to polymerize i) monomers, prepolymers or macromonomers that are water-soluble or form water-soluble sequences with ii) monomers, prepolymers or macromonomers that are water-soluble or capable of forming water-soluble sequences and, either having the LCST property in the useful temperature range, or bearing a side chain having this property.

Operating using polycondensation can also be contemplated.

The invention disclosed in the said application thus relates, generally speaking, to copolymers bearing heatsensitive side chains and synthesis whereof is carried out in accordance with the general principles aforementioned.

In a less preferred manner, the reverse structure will also be contemplated, that is to say a water-soluble polymer chain possessing the LCST property, bearing water-soluble side chains that do not have the LCST property in the temperature range under consideration.

Again in a less preferred manner, copolymerization in sequences that are successively water-soluble with an LCST property and water-soluble without an LCST property is contemplated.

The invention described in the said application has been more particularly developed on the basis:
- of a basic polymer chain or skeleton containing acrylic acid or AMPS (acrylamido-methylpropylsulfonate) residues; and
- heat sensitive grafts of the polyethylene glycol (POE) chain type bearing an active terminal group for grafting purposes, in particular an amine fonction, the POE being replaceable by a POP (propylene polyoxide).

The study has also been extended to other skeletons possessing units of acrylic acid and/or acrylamide and/or AMPS (acrylamido-methylpropylsulfonate) and/or N-methylvinyl N - acetamide.

The description also relates, in particular, to copolymers of AMPS and of POE, POS or POEP based macromonomers. Table A of the said application (Taylor et al., J. of polymer science, 13, 2551-2570 (1975)) gives a non-limitative list of chains with an LCST property in an aqueous solution.

For the purposes of synthesizing the thermoviscosifying polymers according to the invention, the man of the art can implement the conventional, known techniques of copolymerization and grafting.

We shall cite, in particular, the methods given by way of example. The modes of operation, the choice of solvents, etc. for these reactions are well known to a man of the art. Finally, the man of the art can profitably refer to French patent application No. 92 10224 filed on 20th. August 1992 for examples of synthesis.

According to the present invention, it has been discovered that certain of the polymers described in the aforementioned application possessed a remarkable so-called anti-settling property for particules with an order of magnitude of less than a few millimeters.

We synthesized, in accordance with the aforementioned patent application (example 1.2) a copolymer of AMPS and acrylic acid, with POE (polyethylene glycol) grafting.

The molecular weight of the skeleton of the polymer is approximately 700 000 and the ratio of AMPS/ acrylic acid monomers is 80/20 in moles. The molar proportion of grafting by POE is 1.1% POE and the molecular weight of each lateral POE graft is 5000.

The rheological behaviour of this copolymer is represented in the annexed figure, showing log (viscosity) as a function of log (shear rate) at different temperatures. The test was carried out with an apparatus of the "Low Shear LS30" type, the polymer concentration being 3% and the solution containing 15.2% of K₂CO₃.

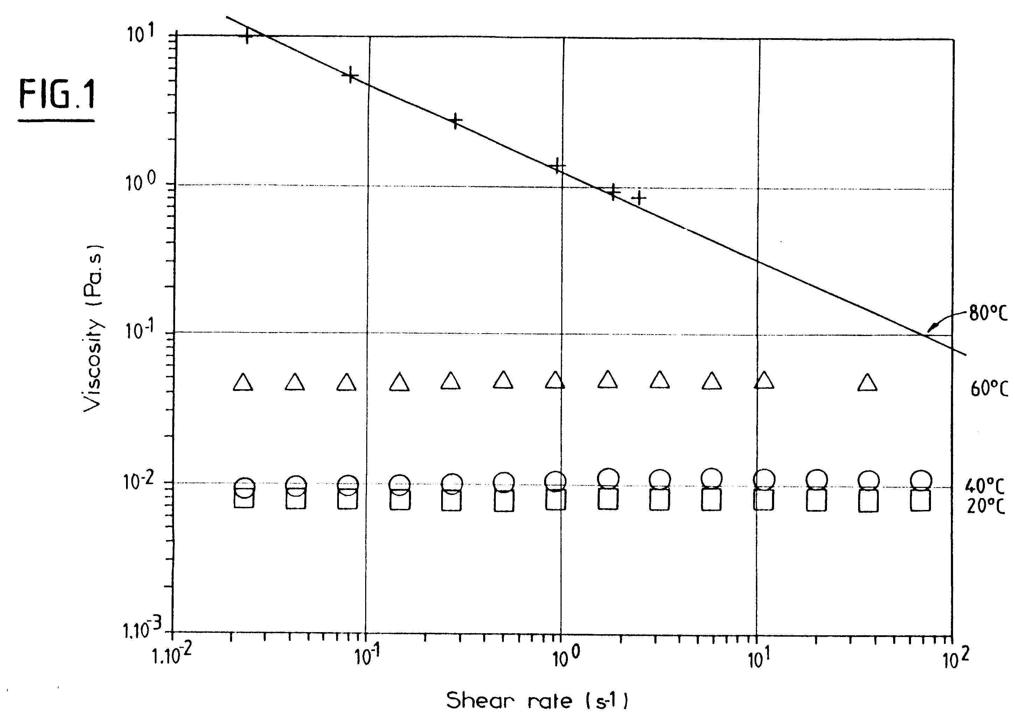

The present invention consists in selecting copolymers belonging to the family described in the aforementioned application, and having a rheological behaviour as represented in annexed figure 1.

This selection will be within the competence of a man of the art upon reading the aforementioned patent application and the present application, in accordance, in particular, with the examples of applications given here.

It is noted that the polymers selected according to the invention exhibit a markedly non-Newtonian behaviour which increases with temperature. On account of this purely thermodynamic aggregation, shearing of their solution can disaggregate them up to a certain point. They have a ratio of viscosity (in the shearing range of approximately 10⁻² to 10¹ s⁻¹) to the shear rate (in the shearing range of approximately 10¹ - 10² s⁻¹) which is then, according to the selection of the invention, highly favorable for creating good anti-settling conditions

A man of the art is, indeed, aware that this ratio largely conditions this property and the ability to preserve good pumpability. This makes it possible, while preserving low viscosity for fluid transport, to prevent the settling that takes place under other shearing conditions.

Another advantage of the invention consists, through the choice or use of temperatures, in opening the way to numerous applications.

These applications are to be found in all industries concerned by the problem of the stability of aqueous suspensions and, in particular, in the oil industry, in which particular-laden fluids are traditionally found which will be subjected to major variations in temperature. Generally speaking, the useful polymers according to the invention will be chosen in such a way that the A/B ratio (with :
A = viscosity in the shear rate range of approximately 10⁻¹ to 10⁻² s⁻¹
and
B viscosity in the shear rate range of approximately 10 to 10² s⁻¹)
is as high as possible in the temperature range of the application.

In log/log representation, as in annexed figure 1, this criterion according to the invention can easily be materialized by the slope of the curve obtained.

The criterion according to the invention resides in the fact that this slope has to be more negative than approximately -0.4 (for example -0.5, 0.6, etc.).

In annexed figure 1, the slope at 80°C is -0.59.

No limit to the negativity of the said slope of the log (viscosity) / log (shear rate) curve has been contemplated, other than the practical limits that will be obvious to a man of the art.

As in the aforementioned patent application, the lateral grafts can be of the PEO, POP, etc. type, and a man of the art will know how to select them in order to refine the properties sought after.

It is important to note that observance of the original criterion hereabove (A/B ratio) and an appropriate choice of the nature and quantity of grafts as a function of the properties sought after (that is to say, in particular, as a function of the temperature range of the application) make it possible to obtain either systems with suspensoid properties that are "permanent" (that is to say the suspensoid property exists over the entire temperature range contemplated), or having a suspensoid property that is a "function of the temperature" (that is to say this property appears above a temperature that can be fixed a priori and that will be a function of the nature of the grafts, of the number of the grafts and of the distribution of their molecular weights).

The major advantage of the invention is that, in systems having a suspensoid property that is a function of temperature, the suspensoid function can appear at a high temperature and disappear at a low temperature.

As this phenomenon is reversible, we can appreciate how valuable this is in industries wherein the laden fluids undergo temperature cycles, in particular the oil industry, and most especially those techniques associated with drilling fluids.

Thus, to constitute cement slurries or separating fluids or "spacers", the general composition and the properties of which are well known, the parameters according to the invention will be selected so as to obtain a fluid having a "permanent" suspensoid property. The advantage of this will be to prevent any untimely settling both at ambient pumping temperature on the surface and at far higher bottom hole temperatures and, when the fluid rises back up to the surface through the annulus, also at the lower temperatures close to surface temperature.

It will be noted that, in the present state of the art, known suspensoid agents have the serious drawback of losing their suspensoid properties when the temperature rises. There is thus a high risk of pumping good quality fluid on the surface, but of finding that very prejudicial settling develops downhole.

In the present state of the art, no really satisfactory solution to this serious problem is known. It will also be noted that, quite surprisingly, the systems according to the invention, as indicated earlier, have precisely the reverse tendency, as their suspensoid activity increases with temperature.

This surprising property will be exploited in the most important application of the systems according to the invention which concerns drilling fluids or muds.

The invention makes it possible, in fact, to design drilling fluids the suspensoid properties of which will be precisely adapted to requirements.

The parameters of the invention will be selected to use a system having suspensoid properties that are a "function of temperature", that is to say which increase with the temperature.

At the surface temperature, the drilling fluid will be pumped normally. At the bottom of the borehole, at a higher temperature, the suspensoid properties will be more marked: the fluid will thus be capable of supporting in suspension the "cuttings" that are known to appear in this area. The fluid will thus be able to bring the cuttings efficiently back up to the surface.

On the other hand, when the mud laden with cuttings is passed through the cutting separation areas ("shakers" or centrifuges), the cooling of the fluid will cause it to lose a sufficient part of its suspensoid properties for the separation of the cuttings to be considerably facilitated.

Finally, thanks to the reversible nature of the phenomenon, conventional "circulation" of the mud through the well is also possible, with the same advantages as heretofore.

It will further be noted that the invention provides a solution to the industrial problems of fouling in aqueous media at a temperature higher than ambient temperature. The addition to the aqueous solution of such polymers makes it possible to avoid the depositing of solid particles in the hot portion of the process and to collect them in the cold portions in order to free the fluid therefrom prior to recycling or discharge of the liquid.

A man of the art will appreciate that the invention applies to all fluids laden with particles the dimension of which is (approximately) less than a few millimeters and, in particular, those that are subjected to cycles or temperature variations and for which control of suspensoid properties is required.

All activities connected with the oil industry, associated industries and industrial purification of fluids are clearly concerned.

Other industries, such as those devoted to cosmetic preparations or food processing, or the lacquer and varnish industry, papermaking and the preparation of suspensions for coating (carbonates, pigments, kaolin, etc.) are also concerned.

The following examples illustrate the invention without, however, limiting the scope thereof.

### Example 1

This involves the example described earlier and corresponding to example 1.2 in the aforementioned French patent application No. 92 10224 filed on 20th. August 1992.

It will be remembered here, to facilitate understanding, that the copolymer involved is one with 92% AMPS and 8% acrylic acid, by weight, modified by 1.1% of POE by weight with lateral grafts having a molecular weight of 5000.

As mentioned earlier, its log (viscosity) / log (shear rate) properties are set forth in annexed figure 1, for a polymer concentration of 3% by weight and a K₂CO₃ content of 15.2%.

### Example 2

As in example 1, a copolymer with 92% AMPS and 8% acrylic acid, by weight, and a molecular weight of 700 000 is prepared.

The rate and other grafting conditions are identical, as is the polymer concentration (3% by weight). On the other hand, the K₂CO₃ concentration is 9.7%.

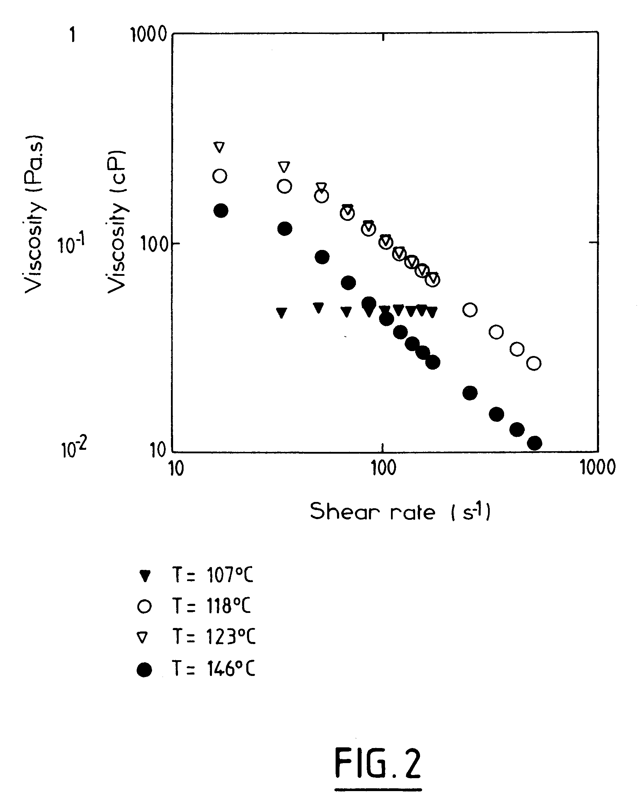

The corresponding log (viscosity / log (shear rate) curve is represented in annexed figure 2.

### Example 3

An AMPS/AA/NMVA terpolymer is synthesized in a manner known in the art as described in example 1.4 of the aforementioned patent application (AMPS 65%; AA 15%; NMVA 20%).

Grafting is carried out at 14% by weight by POE links with a molecular weight of approximately 5000.

A solution with 2% by weight of grafted polymer is prepared.

The K₂CO₃ concentration is 13.8%.

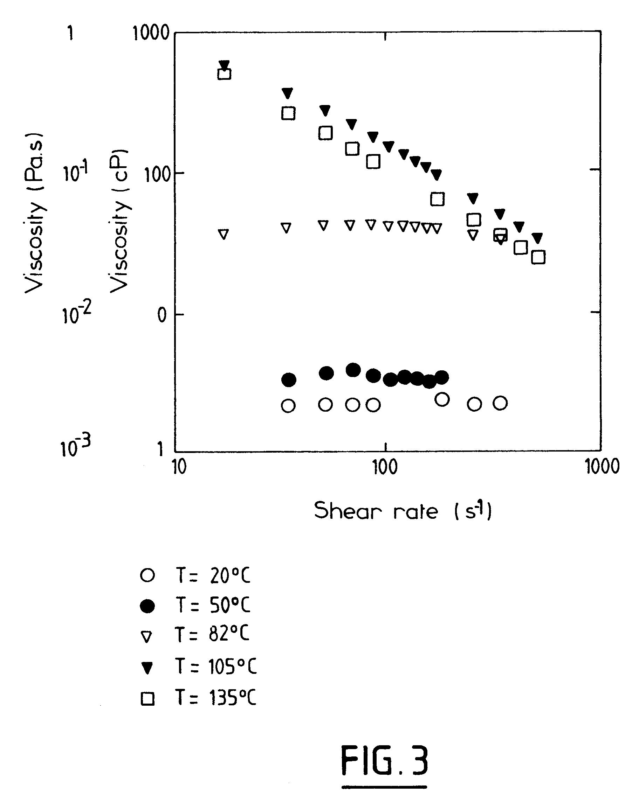

The log (viscosity) / log (shear rate) properties are represented in annexed figure 3.

### Example 4

An acrylic acid polymer with a molecular weight of 500 000 grafted with 0.33% of polyoxyethylene with a molecular weight of approximately 5000 is prepared.

The solution concentration is 2% of grafted polymer. To it is added K₂CO₃ so as to obtain a 1.2 molar solution.

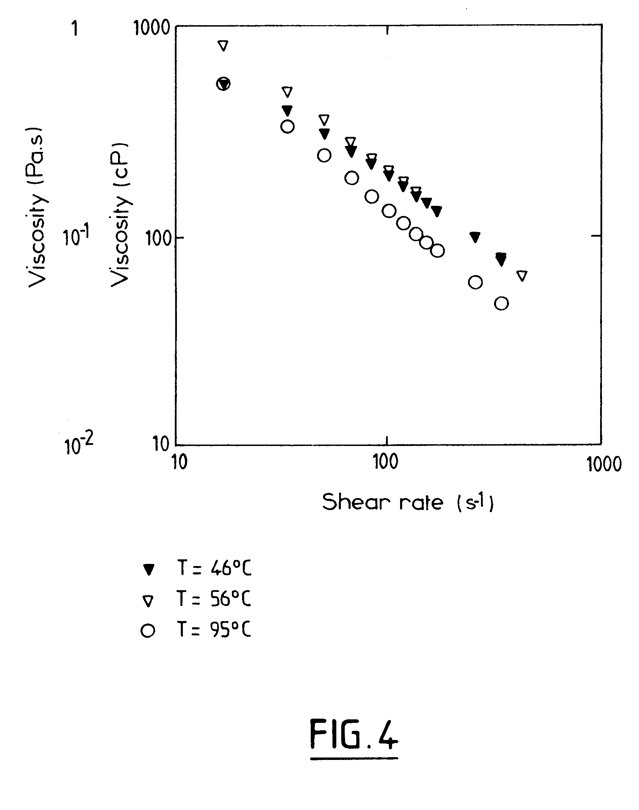

The properties are represented in annexed figure 4.

It can be seen that the polymers corresponding to figures 2, 3 and 4 also satisfy the criteria of the invention.

### Example 5

The following polymers were prepared in a similar manner by grafting lateral chains bearing a grafting active site on corresponding active sites of the skeleton.
- polyacrylic acid with a molecular weight of 500 000 grafted with 0,17 % (molar) of polyethylene oxide of molecular weight 5000.
- polyacrylic acid with a molecular weight of 150 000 grafted with 0,8 % (molar) of polyethylene oxide of molecular weight 5000.
- polyacrylic acid with a molecular weight of 150 000 grafted with 0,4 % (molar) of polyethylene oxide of molecular weight 5000.
- a copolymer pAMPS/2,15-POE5 with a POE molecular weight of 5000.
- a copolymer pAMPS/1,15-POE5 with a POE molecular weight of 5000.
- polyacrylic acid with a molecular weight of 150 000 grafted with 21 % (molar) of polypropylene oxide of molecular weight 600.
- polyacrylic acid with a molecular weight of 150 000 grafted with 5,9 % (molar) of polypropylene and ethylene oxide of molecular weight 1100.
- a terpolymer of AMPS, N-methylvinylacetamide and acrylic acid, grafted with 0,6 % (molar) of POE of moleculr weight 5000.

## Claims

1. Polymers the aqueous solutions of which are of a rheofluidifying nature increasing with the temperature, and which include:
a. chain portions of the hydrophilic prepolymer or macromonomer type not having the LSCT property in the useful temperature range; and
b. chain portions of the hydrophilic prepolymer or macromonomer type having the LCST property in the useful temperature range, characterized in that they are chosen in such a way that the A/B ratio (with:
A = viscosity in the shear rate range of approximately 10⁻¹ to 10⁻² s⁻¹
and
B = viscosity in the shear rate range of approximately 10 to 10² s⁻¹) is as high as possible.

2. Polymers according to claim 1, characterized in that they are chosen in such a way that the slope of the log (viscosity) / log (shear rate) curve is as negative as possible.

3. Polymers according to claim 2, characterized in that the slope of the said curve is more negative than -0.4.

4. Polymers according to claim 3, characterized in that the slope of the said curve is approximately -0.6.

5. Polymers according to any one of claims 1 to 4, characterized in that they consist of a water-soluble polymer skeleton not having the LCST property in the useful temperature range, the said skeleton:
a. bearing grafted hydrophilic side chains having the LCST property in the useful temperature range; or/and:
b. incorporating in the chain sequences consisting of hydrophilic residues having the LCST property in the useful temperature range.

6. Polymers according to claim 5, characterized in that the said chain portions of the prepolymer or macromonomer type or the said hydrophilic skeleton not having the LCST property consist of acrylic chains.

7. Polymers according to claim 6, characterized in that the said hydrophilic skeleton not having the LCST property consists of a terpolymer of acrylic acid (AA), AMPS and Nmethylvinyl acetamide (NMVA).

8. Polymers according to any one of claims 1 to 7, characterized in that the said chain portions of the prepolymer or macromonomer type or the said side chains or hydrophilic sequences having the LCST property consist of POE, POP or POEP chains.

9. Polymers according to any one of claims 1 to 8, characterized in that the said chain portions of the prepolymer or macromonomer type or the said side chains or hydrophilic sequences having the LCST property consist of chains chosen from among:
methylcellulose
hydroxypropylcellulose
polyvinyl alcohol - vinyl acetate copolymers
poly(ethylene glycol)
polypropylene glycol
polymethacrylic acid
poly-N-vinyl-2-oxazolidone
partially acetalized polyvinyl acid
partially cyanoethylated polyvinyl alcohol
poly-N-isopropylacrylamide
poly(vinyl methyl ether)
poly-L-proline
other polyamino acids and proteins
and copolymers made up from hydrophobic and hydrophilic monomers.

10. Polymers according to claim 5, characterized in that they are obtained by grafting the said side chains a. having an active site for grafting onto the corresponding active sites of the said skeleton.

11. Polymers according to claim 5, characterized in that they are obtained by copolymerization of the monomers constituting the said skeleton and the monomers constituting the said sequences according to b. or, in both cases, monomers or prepolymers or macromonomers corresponding, respectively, to the said skeleton and to the said chains.

12. Polymers according to any one of claims 1 to 11, characterized in that they are chosen from among:
- polacrylic acid with a molecular weight of 500 000 grafted with 0.17% (per mole) of ethylene polyoxide with a molecular weight of 5000 obtained using the method of claim 10 ;
- polyacrylic acid with a molecular weight of 150 000 grafted with 0.8% (per mole) of ethylene polyoxide with a molecular weight of 5000, obtained using the method of claim 10 ;
- polyacrylic acid with a molecular weight of 150 000 grafted with 0.4% (per mole) of ethylene polyoxide with a molecular weight of 5000, obtained using the method of claim 10 ;
- a p AMPS/2, 15-POE5 copolymer and a molecular weight of the POE chains of 5000 obtained using the method of claim 11 ;
- a p AMPS/1, 15-POE5 copolymer and a molecular weight of POE chains of 5000, obtained using the method of claim 11;
- polyacrylic acid with a molecular weight of 150 000 grafted with 21% (per mole) of propylene polyoxide with a molecular weight of 600, obtained using the method of claim 10 ;
- polyacrylic acid with a molecular weight of 150 000 grafted with 5.9% (per mole) of propylene polyoxide and ethylene with a molecular weight of 1100, obtained using the method of claim 10 ;
- a terpolymer of AMPS, N-methylvinylacetamide and acrylic acid, grafted with 0.6% (in moles) of ethylene polyoxide with a molecular weight of 5000, obtained using the method of claim 10.

13. Use of polymers according to any one of claims 1 to 12 as anti-settling agents for particles with dimensions of less than a few millimeters, in fracturing fluids or drilling or completion fluids, particularly for the cementing of wells, in the oil and geothermal industries, or for acid well treatment, for mobility control agents or fluids for enhanced oil recovery, and analogous fluids used in the oil industry, such as industrial cleaning fluids, hydraulic fluids and as aqueous base lubricants, in the food processing or cosmetics industry and in any fluid laden with such particles, and, in particular, in those subjected to cycles or to temperature variations.
